# EUROPEAN PATENT APPLICATION

(11) **EP 1 997 487 A1**
(43) Date of publication of application: **03.12.2008**
(21) Application number: 06841729.4
(22) Date of filing: 11.12.2006
(51) Int. Cl.: A61K 31/201, A61K 31/07

(54) **PRODUCT FOR USE IN THE PREVENTION AND TREATMENT OF CARDIOVASCULAR DISEASES, CANCER AND CHRONIC INFLAMMATORY DISEASES**

(30) Priority: 14.12.2005 ES 200503082
(71) Applicant: DIETA MEDITERRÁNEA ACEITES Y VINAGRES, S.A., 41012 Sevilla (ES)
(72) Inventor: MEDINA MONTAÑO, José, 41012 Sevilla (ES)
(74) Representative: Isern-Jara, Nuria
(86) International application number: PCT/ES2006/000677
(87) International publication number: WO 2007/068773

(57) **Abstract**

The invention relates to a product that contains at least lycopene and oleic acid together with antioxidants of natural origin, such as β-carotenes, retinol, phytosterols, tyrosol and fatty acids, which is administered orally and which is specially indicated in the prevention of cardiovascular disease and in pathologies mediated by an anomalous activation of factor NF-ϰB, such as chronic inflammatory diseases and certain types of cancer, including colon cancer and prostate cancer.

## Description

This present invention refers to a new product that contains oleic acid and lycopene which is useful for the prevention and treatment of cancer, cardiovascular diseases and chronic inflammatory diseases.

### BACKGROUND

During the last few years cellular and molecular mechanisms have been identified that regulate the inflammatory processes that take place during atheromatosis, arteriosclerosis and cardiovascular disease. The triggering off of the structural and functional alteration of the arterial wall is the concentration of the oxidised LDL in the plasma and in the arterial wall itself. It is shown that the form of the oxidised LDL is more important in cardiovascular disease than the absolute levels of total HDL and LDL cholesterol. This is due to the way the oxidised LDL acts which is an activator of the cardiovascular inflammatory process.

In the first place the oxidised LDL induces an endothelial alteration that mainly consists of the interruption of the nitric oxide (NO) production that damages the endothelial cells starting a process in which vascular tone is lost and in addition these cells start to express adhesion molecule on their surface that attract the monocytes of the blood to the damaged vascular centre. These monocytes migrate to the inside of the vascular wall phagocyting particles of oxidised LDL cholesterol, activating and transforming it into what we know as "foam cells". The accumulation of these foam cells (atherogenic plaque) is truly damaging to the vascular wall for several reasons, 1) vascular tone is lost, 2) a change is produced in the blood flow that encourages local thromocytes, 3) the foam cells produce inflammatory cytokines (basically interleukin 1 and tumour necrosis Factor, TNFα) that regulates the local inflammation process, and 4) these foam cells produce a series of enzymes known as metalloproteinases that destroy the extracellular core of the connective tissue, and on reducing the amount of collagen, make the atheromatous plaque more fragile, hence the arterial inflammation, additionally makes the breakdown of the plaque easier, and consequently, the appearance of clinical ischemia. A large part of all of these processes are regulated by the activation of the nuclear transcription factor kappa B (NF-KB). (See Libby, Ridker, Maseri. Inflammation and Atherosclerosis. Circulation. (2002);105:1135-1143, Zapolska-Downar. Mononuclear Cell Recruitment and Inflammation in Atherosclerosis. Vascular disease prevention. (2004); 1, 41-51)

The term NF-KB encompasses a family of fully expressed transcription factors, in mammals, that is made up of 5 members: p65 (ReIA), RelB, c-Rel, NF-KB1 (p50 and its precursor p105) and NF-KB2 *(p52 and its* precursor p100). The route of NF-KB is activated by a great variety of inflammatory stimuli that can be inhibited by means of nutracytes (term referred to in this present invention as components food compounds that provide added health benefits) thus validating the use of same in the prevention of not only cardiovascular disease but also in chronic inflammatory processes that go with the malignancy of certain human cancers (for example, of the colon and prostate). See (Siebenlist, Brown, Claudio. Control of lymphocyte development by Nuclear Factor-k8. Nature Reviews. (2005); 5, 435-445, Kumar, Takada, Boriek, Aggarwal. Nuclear factor-kB: its role in health and disease. Journal of Molecular Medicine. (2004); 82:434-448).

Both olive oil and tomato have shown themselves to be two foods with extraordinary value as functional foods and, so, beneficial to health. These health benefits are due to the diverse biological activities that have been clearly shown for the majority of the compounds present in both foods.

The tomato extract is rich in lycopene, phytoene, phytofluene, β-carotene, tocopherols and other bio-active photochemical products. The lycopene, phytofluene and β-carotene are carotenoids present in the diet and are found in a normal way in human serum. Vitamin E in tomato is found as a mixture of α, β, γ and Δ tocopherols. The carotenoids are natural pigments that contribute to the yellow, orange and red pigments of vegetable fibres which are synthesised only by plants, moulds, bacteria and algae and, therefore must be incorporated into the diet.

A large number of epidemiological studies have hinted at an association between the high consumption of vegetables and fruits rich in carotenoids and a reduction of the risk of suffering from cancer. The main organ that is related to the consumption of lycopene is the prostate. Various clinical studies have been conducted in which the effect of lycopene in males has been studied. After a diet rich in carotene, an increase of said compound in the plasma of the subjects was seen. There is proof that this increase in the concentrations of lycopene in the serum and in the tissues is related to a fall in the oxidative stress and the damage to the DNA caused in the prostate cells. A clinical measurement of the possibility of suffering from prostate cancer consists of the degree of the concentration of a specific enzyme, alkaline phosphate in blood. Subjects who were given high intakes of tomato suffered a fall in the blood levels of said enzyme. Normally low levels of alkaline phosphate indicate a better state of health of the prostate tissue. In several clinical studies a lower of incidence of prostate cancer was shown for individuals fed with greater doses of lycopene and thanks to it had greater levels of lycopene in the plasma. The greatest effect of the lycopene was observed in men who frequently consume products made from cooked tomato, such as tomato sauce with oil. In an analysis of the anti-oxidant levels in plasma, only the lycopene was the compound that seemed to have a significant association and consistent with the prevention of prostate cancer. See Wertz, Siler, Goralczyck. Lycopene: modes of action to promote prostate health. Archives of biochemistry and biophysics. (2004); 430(1):127-34, Weisburger. Lycopene and tomato products in health promotion. Experimental biological medicine. (2002); 227:924-927, Campbell, Canene-Adams, Lindshield, Boileau, Clinton, Erdman. Tomato phytochemicals and prostate cancer risk. Journal of Nutrition. (2004); 134: 3486S-3492S, Giovanucci. A review of epidemiologic studies of tomatoes, lycopene, and prostate cancer. Experimental biological medicine. (2002); 227:852-859.

In addition to the anti-carcinogenic effects of lycopene that we have just made reference to, other biological effects have been described brought about by this compound. It has also been shown that the consumption of lycopene is also associated to a lower incidence of cardiovascular disease. Specifically, some scientists have studied the association of lycopene and other carotenoids with the low density lipoproteins (LDL), which play a very important part in the development of arteriosclerosis. Also it has been shown that lycopene inhibits the activation of the NF-KB factor. See (Sesso, Buring, Norkus, Gaziano. Plasma lycopene, other carotenoids, and retinol and the risk of cardiovascular disease in women. American Journal of Clinical Nutrition. (2004); 79:47-53, Panasenko, Sharov, Briviba, Sies. Interaction of peroxynitrite with carotenoids in human low density proteins. Archives of biochemistry and biophysics. (2000); 373: 302-305, Kim, Kim, Ahn, Lee, Moon, Lee, Park. Lycopene suppresses the lipopolysaccharide-induced phenotypic and functional maturation of murine dendritic cells through inhibition of mitogen-activated protein kinases and nuclear factor-KB. Immunology. (2004); 113 203-211*).*

The consumption of olive oil, especially the "extra virgin" quality contributes a series of bio-health benefits to our health, to the point of becoming the most beneficial vegetable fat for human health according to numerous scientific studies.

The olive oil has a raised content of monounsaturated fats, which with normal consumption contribute to the reduction of the cholesterol concentration in the blood. In addition to the fatty acids, olive oil has an unsaponifiable fraction. A very heterogeneous group of substances is being dealt with that are present in low concentrations, and amongst which there are carotenes, squalene and other hydrocarbons, tocopherols, phytosterols, poliphenols and numerous compounds that contribute that contribute to giving the oil its characteristic colour, flavour and aroma. These compounds develop important biological activities, as many of them act as natural vitamins and anti-oxidants and can have effects on the reduction of cholesterol, anti-atherogenics and antiinflammatories. In this sense, the vitamin E content of the extra virgin olive oil must be highlighted as its moderate consumption of oil covers the greater part of the dietary recommendations of this vitamin. See (Welters, Tadayyon, Scarpello, Smith, Morgan. Mono-unsaturated fatty acids protect against β-cell apoptosis induced by saturated fatty acids, serum withdrawal or cytokine exposure. FEBS letters. (2004); 560: 103-108*,* Beauchamp, Keast, More, Lin, Pika, Han, Lee, Smith, Breslin. Ibuprofen-like activity in extra-virgin olive oil. Nature. (2005); 437: 45-46, Kris-Etherton, Pearson, Hargrove, Wan, Moriarty, Fishell, Etherton. High-monounsaturated fatty acid diets lower both plasma cholesterol and triacylglicerol concentrations. American Journal of Clinical Nutrition. (1999); 70:1009-15*,* Owen, Haubner, Würtele, Hull, Spiegelhalder, Bartsch. Olives and olive oil in cancer prevention. European Journal of Cancer Prevention. (2004); 13:319-326, Panagiotakos, Pitsavos, Polychronopoulos, Chrysohoou, Zampelas, Trichopoulo. Can a Mediterranean diet moderate the development and clinical progression of coronary heart disease? A systematic review. Medical Science Monitor. (2004); 10(8): RA193-198).

The effects in the concentration and the composition of the fats in the diet and the taking of cholesterol in the development of cardiovascular coronary disease are the basis of the lipid hypothesis of the ateriosclerosis. Therefore, the concentration of the diet is not only related to the concentration of circulating lipids and lipoproteins, but also with the incidence and prevalence of cardiovascular disease in the population. The type and the density of the LDL, in conjunction with the genetics and the diet of each individual, must play an important role in the risk of a person suffering from coronary disease. Another advantageous property of the monounsaturated fatty acids is its power against the oxidation of the LDL lipoproteins, a fact that has been fully demonstrated. The oxidation of the LDL is probably an important factor in atherogenesis as the LDL particles cannot be recognised by the LDL receptor and then, they are associated to some macrophage receivers, in such a way that these accumulate the cholesterol leading to the formation of foam cells and fatty sections. These events bring about the development of atherosclerosis. The monounsaturated fatty acids, thanks to their chemical structure, are much more stable and have less propensity to lipid peroxidation than the polyunsaturated fatty acids (more unstable). This means, a high intake of monounsaturated fatty acids leads us to an increase of the concentration of these fatty acids in the LDL particles. This leads to a lower susceptibility of these particles to the modification of oxidation in *vitro,* when we compare them with LDL particles that contain high concentrations of unsaturated fatty acids (the consequence of a diet that is rich in these fatty acids). See (Wahrburg. What are the health effects of fat? European Journal of Nutrition. (2004); 43: I/6- I/11, Monserrat Fitó Colomer. *The anti-oxidant effects of olive oil and its phenolic compounds.* 2003. Doctoral Thesis).

Olives and olive oil contain high concentrations of phenolic anti-oxidants, squalene and vitamin E. Some studies have shown the relationship between the consumption of foods rich in vitamin E and a lower mortality rate from cardiovascular disease.

The *hydroxytyrosol* forms part of the phenolic compounds of the olive oil. Its ingestion has been seen by part of the organism, which is essential to show its activity. This compound presents a strong anti-oxidant activity, that protects the LDL from being oxidised. This anti-oxidant activity is even greater than that of the tocopherols or carotenes. See (Tuck, Hayball. Major phenolic compounds in olive oil: metabolism and health effects. Journal of Nutritional Biochemistry. (2002); 13(11):636-644, Visioli, Poli, Galli. Antioxidant and other biological activities of phenols from olives and olive oil. Medicinal Research Reviews. (2002); 22(1):65-75, Mateos, Dominguez, Espartero, Cert. Antioxidant effect of phenolic compounds, α tocopherol, and other minor components in virgin olive oil. Journal of Agricultural and Food Chemistry. (2003); 51(24):7170-5, Puerta, Dominguez, Gutiérrez, Flavill, Hoult. Effects of virgin olive phenolics on scaven ging of reactive nitrogen species and upon synergic neurotransmission.Life Sciences. (2001); 69(10):1213-22, Rune, Blomhoff. Dietary antioxidants and cardiovascular disease. Current Opinion in Lipidology. (2005); 16(1):47-54).

Unlike the already known products in the technique, the present invention provides a product that mainly includes, oleic acid and lycopene, together with other natural anti-oxidants, and that has the following advantages: (a) as a result of its anti-oxidant capacity it can regulate the oxidation of the LDL that bring on the development of the cardiovascular disease; (b) due to the presence of inhibiting compounds of the NF-kB transcription factor it can regulate inflammatory phenomena, which are related to onset and development of differing chronic pathologies such as cardiovascular disease, rheumatoid arthritis, and the inflammation associated with tumours; (c) thanks to containing compounds that are potentially anti-carcinogenic, it can prevent, reduce or slow down the risk of cancer of differing aetiologies; (d) due to the presence of natural anti-oxidants a synergic effect is produced; (e) it is easy to prepare and (f) it has a pleasant flavour for the palate. Said advantages can be found individualised in different products, but there is no product as such that encompasses all of these characteristics.

### DETAILED DESCRIPTION OF THE INVENTION

One aspect of the present invention makes reference to a new nutraceutical product that includes therapeutically effective amounts of oleic acid (monounsaturated fatty acid with regulatory properties for the inflammatory response) and lycopene (carotene with preventive activity of the cardiovascular disease), that are used together with suitable amounts of one or more of the following compounds: (a) β-carotene (mainly coming from tomato and olive oil and the precursor of vitamin A with a significant anti-oxidant capacity) and/or an equimolar amount of its derivatives, preferably such as retinol (vitamin A) and derivatives of same ; (b) retinol (mainly coming from tomato and olive oil) and all of the derivatives from the (bio)chemical action, preferably, the retinol isomers all-trans and 11-cis retinol, the oxidation products retinoic acid all-trans, acid 13-cis retinoic, acid 9-cis retinoic and the 11-cis retinal reduction product, together with all the derivatives of same; (c) free fatty acids (mainly coming from olive oil) act as the regulators of the metabolism of the LDL) such as linoleic acid, linolenic acid, palmitic acid, stearic acid, arachidonic acid, palmitoleic acid, gadoleic acid and behenic acid, likewise the derivatives from the (de)saturation of same, preferably the palmitic, stearic, oleic, linoleic, linolenic and arachidonic acids; (d) phytosterols (compounds belonging to the unsaponifiable fraction of olive oil that combines with a wide range of anti-oxidant biological, anticarcinogenic and anti atherogenic activities), such as β-sitosterol, camesterol, stigmasterol, cicloartenol, sitostanol, campestanol, preferably β-sitosterol, or equimolar concentrations of its derivatives (combinations of free fatty acids forming esters) and their saturation products, such as the tocopherols (they can act reducing the risk of suffering from cardiovascular disease) together with the isomers α-vitamin E, β and γ and other tocotrienols; (e) tirosol (phenyl acetic acid derived from tyrosine ([2-(4-hidroxiphenyl) ethanol]) belonging to the unsaonifiable fraction of the olive oil) and its derivatives coming from hydrogenation, such as hydroxtyrosol [2-(3,4-dihydroxyphenyl) ethanol].

The addition of these natural anti-oxidants to the initial mixture made up of lycopene and oleic acid gives a n effect that can be added to the anti-oxidant activity that would be provided if each one were performed in an individual manner.

In a preferred embodiment of this present invention a compound is provided that is prepared by means of mixture and homogenisation, that is given orally and a 20 ml. volume contains:
- Oleic Acid in an amount that varies between 1 and 15 g.
- Lycopene in an amount that varies between 1 and 125 mg.
- β-carotene in an amount that varies between 0.1 and 10 mg.
- Linoleic Acid in an amount that varies between 0.1 and 10 g.
- Palmitoleic Acid in an amount that varies between 0.01 and 2 g.
- Phytosterols in an amount that varies between 1 and 100 mg.
- α-Tocopherol (vitamin-E) in an amount that varies between 0.1 and 10 mg.
- Tyrosol derivative, preferably hydroxytyrosol-tyrosol in an amount between 0.01 and 1 mg.

Another aspect of the present invention makes reference to the use of the present compound as:
(a) anti-oxidant, as it can regulate the oxidation of the LDL that bring about the development of cardiovascular disease;
(b) regulator of the inflammatory processes due to the presence of NF-kB transcription factor inhibitor compounds, where said processes are related to the appearance and development of differing chronic pathologies such as cardiovascular disease, rheumatoid arthritis, and the inflammation associated with tumours.
(c) potentially anti carcinogenic, as it can prevent, reduce or delay the risk of cancer of differing aetiologies.

### EXAMPLES

The following examples are not by way of limitation and illustrate the invention for a compound of tomato extract, olive oil and a mixture of olive oil and tomato extract.

### EXAMPLE 1: Inhibition of the activation of the kappa B (NF-kB) transcription factor by TNFά with a tomato extract that contains a minimum of 6% lycopene in linfoid cells.

### METHOD

Cells. A cellular line 5.1, that is a clone derived from Jurkat stably transfected with a plasmid that contains the gene of the luciferase channelled by the HIV-LTR promoter, an exponential growth is maintained in a RPMI 1640 medium supplemented with a 10% of an foetal bovine serum deactivated by heat, 2 mM of L-glutamine, 1 mM HEPES, antibiotics and 200 µg/ml of G418.

Luciferase Trial. The 5.1 cellular line is pre-incubated for 30 minutes with tomato extract that had a minimum of 6% lycopene, followed by stimulation with TNFá (2ng/ml) for 6 hours. Next, the cells were damaged in 25 mM tris-phosphate pH 7,8, 8 mM MgCl₂ 1 mM DTT, 1 % Triton X-100, y 7% glycerol. The luciferase activity was measured using Autolumat LB 9501 following the instructions of the luciferase trial kit and the protein concentration was measured by the Bradford method. In order eliminate the background noise caused by the lysis solution, this value was deducted in each value of the experiment and the activation was expressed as proportional induction compared to the untreated cells.

Composition of the extract: The tomato extract used for the trial mainly contains: lycopene (6%), phytoene (0.6%), phytofluene (0.5%), β-carotene (0.2%), tocopherols (2%), phytosterols (0.6%).

### RESULT

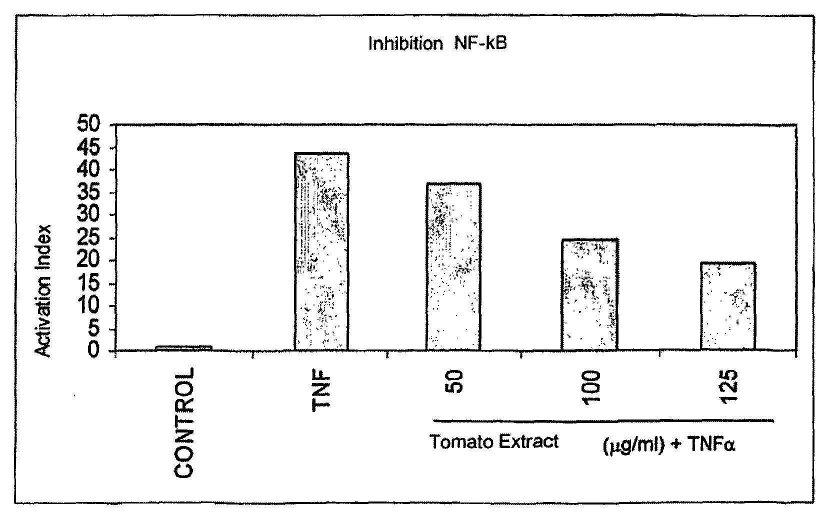

### EXAMPLE 2: Inhibition of the DNA bond of NF-kB by TNFά with tomato extract that contains a minimum of 6% lycopene in linfoid cells.

### METHOD

Delayed trial in gel. Isolation of proteins and electrophoretic mobility trial (EMSA) The cells were stimulated 5.1(10⁶/ml) in a complete medium as previously stated with tomato extract with a minimum of 6% lycopene. Next, the cells were washed twice with a cold saline phosphate tampon, the proteins from the nuclear extracts were isolated and the concentration of the proteins were determined using the Bradford method (Bio-rad). For the electrophoretic mobility trial (EMSA) the agreed sensor of NF-kB 5'-AGTTGAGGGGACTTTCCCAGG-3' was marked with the line [γ-³²P]ATP. The mixture for the reaction of the union contains 3 µg of the proteic extract, 0.5 µg de poly(dl-dC), 20 mM HEPES, pH 7,70 mM NaCl, 2 mM DTT, 0.01 % NP-40, 100 µg/ml BSA, 4% Ficoll, and 100,000 cpm of DNA fragments marked in the line in a total volume of 20 µl. After 30 minutes incubation at 4°C, the mixture was subjected to electrophoresis in 6% polyacrylamide gel that contained 89 mM Tris-borate, 89 mMboric acid and 1 mM EDTA. The gels were previously subjected to electrophoresis at 225V for 30 minutes and then for 2 hours after the samples were loaded. These gels were dried and exposed to an X-ray film at -80° C.

### RESULT

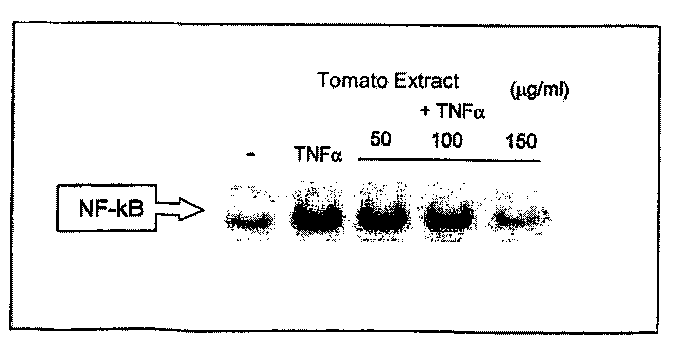

### EXAMPLE 3: Inhibition of the NF*kB activation by TNFά with oleic acid in linfoid cells.

### METHOD

Cells. The 5.1 cellular line was used, as and how described in example 1.

Luciferase Trial. The 5.1 cellular line was pre-incubated for 30 minutes with oleic acid in the concentrations as stated, followed by stimulation with TNFά (2 ng/ml) for 6 hours. The NF-kB transcriptional activity was measured in the same way as in example 1.

### RESULT

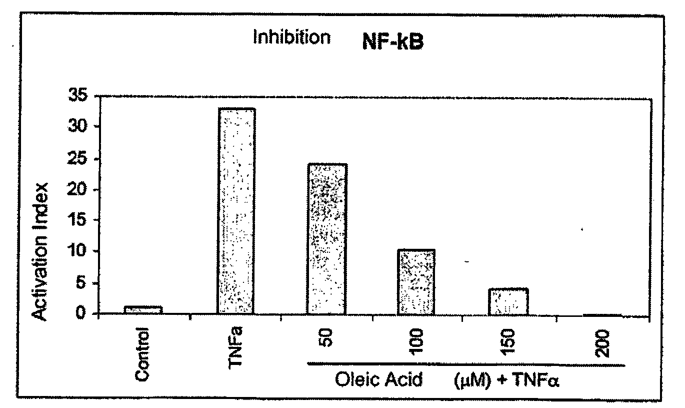

### EXAMPLE 4:

### Inhibition of the NF-kB activation by TNFα with oleic acid in lung cancer cells.

### METHOD

Cells. The A549 cellular line of adenocarcinoma of the lung was maintained in the DMEM *(Dulbecco's modified* Eagle's) medium, complemented with 10% of foetal bovine serum that has been deactivated by heat.

Transitory *transfections and luciferase trial.* The A549 (10⁵/ml) cells were transfected with LTR-Luc reporter gene and with KBF-Luc. The transfections were carried out using the LipofectAMINE PLUS transfection agent, according to the instructions from the manufacturer, for 24 hours. After the incubation with oleic acid for 30 minutes, the transfected cells were stimulated for 6 hours with TNFά (2µg/ml). The NF-kB transcriptional activity was measured in the same way as in example 1.

### RESULT

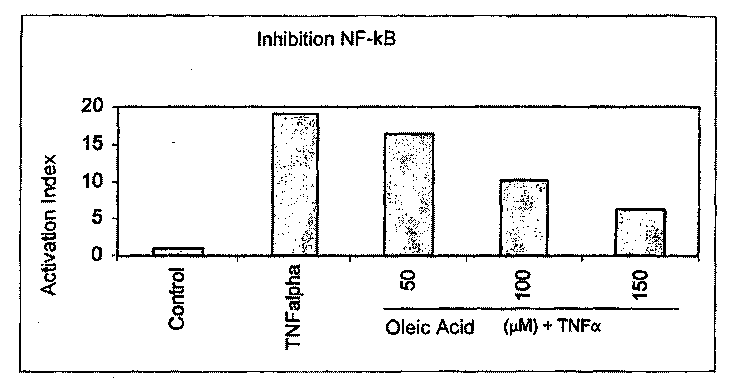

### EXAMPLE 5: Inhibition of the NF-kB activation by TNFά with the combination of oleic acid and lycopene.

### METHOD

Cells. The 5.1 cellular line was used, as and how described in example 1.

Luciferase Trial. The 5.1 cellular line was pre-incubated for 30 minutes with oleic acid in the presence of lycopene, followed by stimulation with TNFα (2 ng/ml) for 6 hours. The NF-kB transcriptional activity was measured in the same way as in example 1.

### RESULT

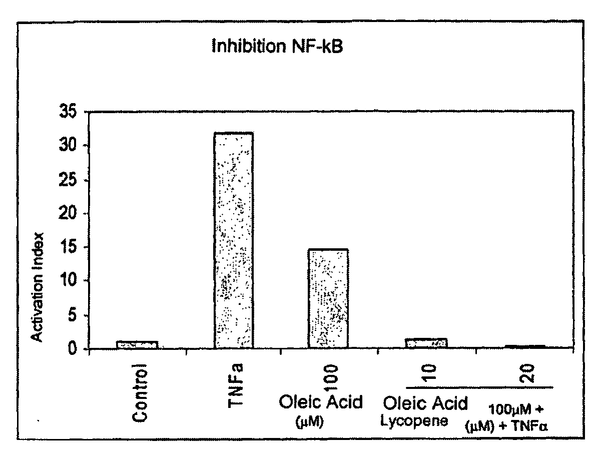

In the light of the results it can be seen that both the lycopene from the tomato extract and equally the oleic acid from olive oil offer individual optimal results in the inhibition of the NF-kB, transcription factor activation, specifically, in example 1 with tomato extract for linfoid cells, the activation index reduces to approximately 25 when the concentration increases to 125 µg/ml, whilst in example 3 with oleic acid with the same concentration for linfoid cells, the activation index reduces to approximately, 7-8.

In example 2, a lesser amount of NF-kB joined to the DNA is observed on increasing the concentration of the tomato extract.

In example 4, a similar effect to that in example 3 is seen even though in this case it is acting on cancer cells.

Finally, example 5 uses an oleic acid and lycopene mix varying the concentrations of lycopene (10 µM y 20 µM). It can be clearly seen that there is a synergic effect as the activation index reduced to values below 5. In addition, the greater the lycopene concentration the lower the index.

In conclusion, it is clear that the combination of both components (lycopene and oleic acid) produces a greater effect on the inhibition of the NF-kB transcription factor activation, which is involved in the processes related to appearance and development of chronic pathologies, such as cardiovascular disease, rheumatoid arthritis and the inflammation associated with tumours.

## Claims

1. A product that includes a therapeutically effective amount of lycopene and a therapeutically effective amount of oleic acid and suitable amounts of one or more of the following compounds:
(a) β-carotene and/or an equimolar amount of its derivatives, such as retinol (vitamin A) and derivatives of same;
(b) retinol and all of its derivatives by (bio)chemical action, preferably, its retinol isomers all-trans and 11-cis retinol, the retinoic acid oxidation products all-trans, acid 13-cis retinoic, acid 9-cis retinoic and the reduction product 11-cis retinal, together with all of the derivatives of same;
(c) free fatty acids, such as linoleic, linolenic, palmitic, stearic, arachidonic, palmitoleic, gadoleic and behenic acids, likewise the derivatives by (de) saturation of same, preferably, palmitic, stearic, oleic, linoleic, linolenic, arachidonic acids;
(d) phytosterols, such as β-sitosterol, camesterol, stigmasterol, cycloartenol, sitostanol, campestanol, preferably β-sitosterol, or equimolar concentrations of their derivatives (combinations of free fatty acids forming esters) and their products by saturation, such as the tocopherols together with their isomers ά-vitamin E-, β and γ and other tocotrienols;
(e) tyrosol and its derivatives coming from hydrogenation, such as hydroxytirosol [2-(3,4-dihydroxphenyl) ethanol).

2. Product, according to Claim 1, that by preference includes: oleic acid (1-15 g), palmitoleic acid (0.01-2 g), lycopene (1-125 mg), β-carotene (0.1-10 mg), linoleic acid (0.1-10g), phytosterols (1-100 mg), ά-tocopherol (0.1-10 mg), derived from tirosol (0.01-1 mg).

3. Product, according to Claims 1 and 2, in which, as a minimum, the oleic acid acts as an anti-oxidant.

4. Product, according to Claims 1 and 2, in which, as a minimum, the oleic acid acts as a NF-kB inhibitor.

5. Product, according to Claims 1 and 2, in which, as a minimum, the lycopene acts as an anti-oxidant.

6. Product, according to Claims 1 and 2, in which, as a minimum, the lycopene acts as a NF-kB inhibitor.

7. Product, according to Claims 1 and 2, in which, as a minimum, the linoleic acid acts as an additional anti-oxidant.

8. Product, according to Claims 1 and 2, in which, as a minimum, the palmitoleic acid acts as an additional anti-oxidant.

9. Product, according to Claims 1 and 2, in which, as a minimum, the ά-tocopherol acts as an additional anti-oxidant.

10. Product, according to Claims 1 and 2, in which, as a minimum, the tyrosol derivative acts as an additional anti-oxidant.

11. Product, according to Claims 1 and 2, in which, as a minimum, the phytosterols act as additional anti-oxidants.

12. The use of a combination of lycopene and oleic acid together with one or more of the following compounds:
(a) β-carotene and/or an equimolar amount of its derivatives, such as retinol (vitamin A) and derivatives of same;
(b) retinol and all of its derivatives by (bio)chemical action, preferably, its retinol isomers all-trans and 11-cis retinol, the retinoic acid oxidation products all-trans, acid 13-cis retinoic, acid 9-cis retinoic and the reduction product 11-cis retinal, together with all of the derivatives of same;
(c) free fatty acids, such as linoleic, linolenic, palmitic, stearic, arachidonic, palmitoleic, gadoleic and behenic acids, likewise the derivatives by (de) saturation of same, preferably, palmitic, stearic, oleic, linoleic, linolenic, arachidonic acids;
(d) phytosterols, such as β-sitosterol, camesterol, stigmasterol, cycloartenol, sitostanol, campestanol, preferably β-sitosterol, or equimolar concentrations of their derivatives (combinations of free fatty acids forming esters) and their products by saturation, such as the tocopherols together with their isomers ά-vitamin E-, β and γ and other tocotrienols;
(e) tyrosol and its derivatives coming from hydrogenation, such as hydroxytirosol [2-(3,4-dihydroxphenyl) ethanol).
for the preparation of a new product that is administered orally and acts as:
(a) anti-oxidant, as it can regulate the oxidation of the LDL that bring about the development of the disease;
(b) regulator of the inflammatory processes due to the presence of the NF-kB transcription factor inhibitor compounds, where said processes are related to the appearance and the development of differing chronic pathologies, such as cardiovascular disease, rheumatoid arthritis, and inflammation associated with tumours;
(c) potentially anti carcinogenic, as it can prevent, reduce or delay the risk of cancer of differing aetiologies.
